# EUROPEAN PATENT APPLICATION

(11) **EP 4 386 762 A1**
(43) Date of publication of application: **19.06.2024**
(21) Application number: 22855831.8
(22) Date of filing: 04.08.2022
(51) Int. Cl.: G16B 30/00

(54) **INFORMATION PROCESSING SYSTEM AND INFORMATION PROCESSING METHOD**

(30) Priority: 10.08.2021 JP 2021130423
(71) Applicant: NEC Corporation, 108-8001 Tokyo (JP)
(72) Inventor: TAKAHASHI, Shingo, Tokyo 108-8001 (JP); TANAKA, Yuki, Tokyo 108-8001 (JP); ONOUE, Kousuke, Tokyo 108-8001 (JP)
(74) Representative: Betten & Resch
(86) International application number: PCT/JP2022/029879
(87) International publication number: WO 2023/017768

(57) **Abstract**

An information processing system has: a first diagnosis unit that diagnoses quality of a sequence of first fragmented DNA/RNA data, which a first sequencer has output for a sample extracted from a tissue of a target patient, using quality data output from the first sequencer, and determines at least one first diagnosis data indicating a diagnosis result; a second diagnosis unit that diagnoses quality of a sequence of second fragmented DNA/RNA data, which a second sequencer has output for the sample extracted from the tissue of the target patient, using quality data output from the second sequencer, and determines at least one second diagnosis data indicating a diagnosis result; and an output unit that outputs the synthesis DNA/RNA data from the first fragmented DNA/RNA data and the second fragmented DNA/RNA data based on the first diagnosis data and the second diagnosis data.

## Description

### Technical Field

The present invention relates to an information processing system and an information processing method.

### Background Art

Heretofore, existing medicinal drugs have been manufactured in advance and stored in a pharmacy, and prescribed to a patient according to a doctor's prescription. Recently, a patient has come to be dosed with medicines (hereinafter referred to as personalized medicines) that are suitable for the patient's physical constitution or disease characteristics.

Thus far, treatments and medicines for cancer medical care have been selected for each type of cancer such as lung cancer, colorectal cancer, and breast cancer. However, in the 2000s, a molecule (protein) causing cancer and a gene serving as the base of the molecule have become elucidated step by step, and a "molecular target medicine" acting on such a molecule, gene, and the like has become available. Further, as a more developed form of the molecular target medicine, a treatment for each patient's molecules or genes has been developed. Here, a treatment suitable for each person according to not only the type of cancer but also the characteristics of cancer such as a genetic mutation is referred to as a "personalized treatment." Heretofore, a "personalized treatment" based on cancer gene information has been performed mainly on the basis of "cancer gene examination" for examining a small number of genes and "cancer gene panel examination" for examining a large number of genes at the same time.

Recently, a treatment (hereinafter referred to as a cancer vaccine treatment) using cancer immunotherapy for "attacking and eliminating cancer cells by the power of human's inherent immunity" has further progressed. A "peptide vaccine" is known as one of cancer vaccine treatments. A peptide vaccine contains antigens that are markers of cancer. When the antigens are directly injected into a human body, an immune function that a human originally has detects abnormality and attacks against the antigens that are markers of cancer as a target to kill cancer cells.

Recently, all cancer genetic mutations of each patient have become easily known, and the importance of "peptide" newly generated by the genetic mutations has been highlighted in cancer immunotherapy. This peptide is a mutated peptide called "neoantigen." It has become clear that when these peptides come out on the surface of cancer cells by getting on human leukocyte antigens (HLAs), cytotoxic T lymphocytes (CTLs) can regard them as an enemy and kill the cancer cells because they are peptides not observed on the surface of normal cells.

Currently, clinical trials are being conducted in Europe and the United States to confirm whether a personalized cancer vaccine therapy using "neoantigens" is effective as a cancer treatment or recurrence prevention method. In addition, Patent Literature 1 discloses a method for identifying a fragment of a polypeptide that is immunogenic to a specific human subject and a method for preparing a personalized pharmaceutical composition containing the polypeptide fragment.

### Citation List

### Patent Literature

Patent Literature 1: JP 2020-510698 A

### Summary of Invention

### Technical Problem

Since the type and number of mutated peptides called "neoantigens" vary from one patient to another, it is necessary to produce a peptide vaccine individually for each patient from genetic mutations of the patient's cancer tissues. It is necessary to deliver the peptide vaccine to the patient before the disease of the patient progresses; however, since this product is produced on orders, if there is a mistake even in one process among multiple processes, it is necessary to repeat the process again, and thus there is a problem that the provision to the patient is delayed.

In a process of producing the peptide vaccine, fragmented DeoxyriboNucleic Acid (DNA) data is output through a pre-analysis process and an analysis process before a vaccine recipe is created. Here, the pre-analysis process includes, for example, specimen collection, formalin fixation, preparation of a pathological tissue specimen, confirmation of a tumor cell ratio, determination of a pathological tissue specimen for genetic testing, and extraction of nucleic acid from the determined specimen. The analysis process includes, for example, library adjustment, replication by Polymerase Chain Reaction (PCR), sequencing, and data output. Data output from a sequencer is a collection of data that is affected by fragmentation performed in the library adjustment and obtained by collecting multiple fragments of DNA/RNA data.

The data output from the sequencer is not constant due to the influence of the pre-analysis process and the analysis process. The output data is affected particularly by the quality of an input DNA sample, the variation in the PCR process, and the accuracy of sequencing. Normally, the quality of the output data is ensured by performing validation, internal accuracy control, and external accuracy control based on quality control standards provided for each process. However, the poor quality may be found at the end of the process; therefore, there is a problem that, when the process needs to be restarted, the vaccine production is delayed and the provision to the patient is delayed.

Note that, not only cancer vaccines but also autoimmune diseases may be treated with similar neoantigens, and therefore there is a similar problem in personalized medical care of autoimmune diseases.

The present invention has been made in view of the above problem, and aims to provide an information processing system and an information processing method capable of suppressing delay in manufacturing of a vaccine for personalized medical care.

### Solution to Problem

An information processing system according to a first aspect of the present invention is an information processing system that outputs synthesis DNA/RNA data used for synthesizing a mutated peptide derived from a genetic mutation of cancer, the information processing system including: a first diagnosis unit that diagnoses quality of a sequence of first fragmented DNA/RNA data, which a first sequencer has output for a sample extracted from a tissue of a target patient, using quality data output from the first sequencer, and determines at least one first diagnosis data indicating a diagnosis result; a second diagnosis unit that diagnoses quality of a sequence of second fragmented DNA/RNA data, which a second sequencer has output for the sample extracted from the tissue of the target patient, using quality data output from the second sequencer, and determines at least one second diagnosis data indicating a diagnosis result; and an output unit that outputs the synthesis DNA/RNA data from the first fragmented DNA/RNA data and the second fragmented DNA/RNA data based on the first diagnosis data and the second diagnosis data.

This configuration increases the possibility of outputting high-quality synthesis DNA/RNA data for synthesizing a mutated peptide, and suppresses repetition of the process due to deterioration in quality of synthesis DNA/RNA data, so that delay in manufacturing of a personalized cancer vaccine can be suppressed.

An information processing system according to a second aspect of the present invention is the information processing system according to the first aspect, in which the first diagnosis unit may diagnose the quality of the sequence of the first fragmented DNA/RNA data using quality information obtained by a pre-analysis process, performed before the sequence of the first sequencer, in addition to the quality data output from the first sequencer, and determine the first diagnosis data, and the second diagnosis unit may diagnose the quality of the sequence of the second fragmented DNA/RNA data using quality information obtained by a pre-analysis process, performed before the sequence of the second sequencer, in addition to the quality data output from the second sequencer, and determine the second diagnosis data.

An information processing system according to a third aspect of the present invention is the information processing system according to the first or second aspect, in which the output unit may switch an output between the first fragmented DNA/RNA data and the second fragmented DNA/RNA data based on the first diagnosis data and the second diagnosis data, and output the selected output as the synthesis DNA/RNA data.

An information processing system according to a fourth aspect of the present invention is the information processing system according to the first or second aspect, in which the output unit may combine a part of the first fragmented DNA/RNA data and a part of the second fragmented DNA/RNA data based on the first diagnosis data and the second diagnosis data, and output the combined data as the synthesis DNA/RNA data.

An information processing system according to a fifth aspect of the present invention is an information processing system that outputs synthesis DNA/RNA data used for synthesizing a mutated peptide derived from a genetic mutation of cancer, the information processing system comprising an output unit that couples first fragmented DNA/RNA data, which a first sequencer has output for a sample extracted from a tissue of a target patient, to second fragmented DNA/RNA data, which a second sequencer has output for the sample extracted from the tissue of the target patient, and outputs the coupled data as the DNA/RNA data.

An information processing system according to a sixth aspect of the present invention is the information processing system according to the fifth aspect, in which, in a case where outputting the synthesis DNA/RNA data, the output unit may output diagnosis data including the order of priority of the mutated peptide together with the synthesis DNA/RNA data.

An information processing system according to a seventh aspect of the present invention is an information processing system that outputs synthesis DNA/RNA data for synthesizing a mutated peptide derived from a genetic mutation of cancer, the information processing system including an output unit that determines the synthesis DNA/RNA data by determining bases of the respective orders by carrying out a majority decision between bases of the corresponding order of first fragmented DNA/RNA data which a first sequencer has output for a sample extracted from a tissue of a target patient, second fragmented DNA/RNA data which a second sequencer has output for the sample extracted from the tissue of the target patient, and third fragmented DNA/RNA data which a third sequencer has output for the sample extracted from the tissue of the target patient.

An information processing method according to an eighth aspect of the present invention is an information processing method of outputting synthesis DNA/RNA data for synthesizing a mutated peptide derived from a genetic mutation of cancer, the information processing method including: diagnosing quality of a sequence of first fragmented DNA/RNA data, which a first sequencer has output for a sample extracted from a tissue of a target patient, using quality data output from the first sequencer, and determining at least one first diagnosis data indicating a diagnosis result; diagnosing quality of a sequence of second fragmented DNA/RNA data, which a second sequencer has output for the sample extracted from the tissue of the target patient, using quality data output from the second sequencer, and determining at least one second diagnosis data indicating a diagnosis result; and outputting the synthesis DNA/RNA data from the first fragmented DNA/RNA data and the second fragmented DNA/RNA data based on the first diagnosis data and the second diagnosis data.

An information processing method according to a ninth aspect of the present invention is an information processing method of outputting synthesis DNA/RNA data for synthesizing a mutated peptide derived from a genetic mutation of cancer, the information processing method including: coupling first fragmented DNA/RNA data, which a first sequencer has output for a sample extracted from a tissue of a target patient, to second fragmented DNA/RNA data, which a second sequencer has output for the sample extracted from the tissue of the target patient, and outputting the coupled data as the DNA/RNA data.

An information processing method according to a tenth aspect of the present invention is an information processing method of outputting synthesis DNA/RNA data used for synthesizing a mutated peptide derived from a genetic mutation of cancer, the information processing method including: determining the synthesis DNA/RNA data by determining bases of the respective orders by carrying out a majority decision between bases of the corresponding order of first fragmented DNA/RNA data which a first sequencer has output for a sample extracted from a tissue of a target patient, second fragmented DNA/RNA data which a second sequencer has output for the sample extracted from the tissue of the target patient, and third fragmented DNA/RNA data which a third sequencer has output for the sample extracted from the tissue of the target patient.

### Advantageous Effects of Invention

One aspect of the present invention increases the possibility of outputting high-quality synthesis DNA/RNA data for synthesizing a mutated peptide, and suppresses repetition of the process due to deterioration in quality of synthesis DNA/RNA data, so that delay in manufacturing of a personalized cancer vaccine can be suppressed.

### Brief Description of Drawings

Fig. 1 is a schematic diagram illustrating an example of a manufacturing process of a personalized cancer vaccine.
Fig. 2 is a schematic block diagram of an information processing system according to a first embodiment.
Fig. 3A is a schematic diagram illustrating processing of an output unit according to the first embodiment.
Fig. 3B is a schematic diagram illustrating processing of the output unit according to a first modification of the first embodiment.
Fig. 4 is a schematic block diagram of the information processing system according to a second modification of the first embodiment.
Fig. 5 is a schematic block diagram of an information processing system according to a second embodiment.
Fig. 6 is a schematic block diagram of an information processing system according to a third embodiment.

### Description of Embodiments

Hereinafter, each embodiment will be described with reference to the drawings. However, an unnecessarily detailed description may be omitted. For example, a detailed description of a well-known matter and a repeated description of substantially the same configuration may be omitted. This is to avoid unnecessary redundancy of the following description and to facilitate understanding of those skilled in the art.

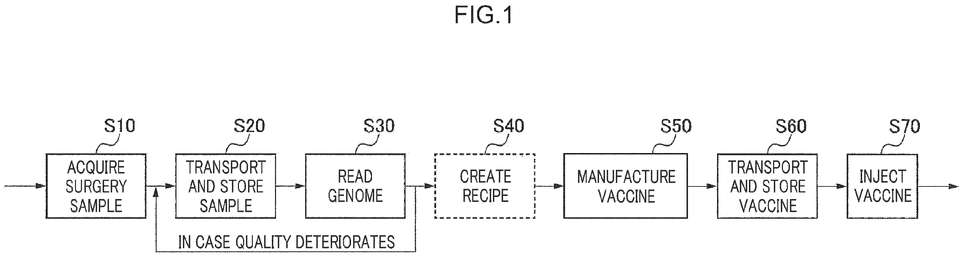

Fig. 1 is a schematic diagram illustrating an example of a manufacturing process of a personalized cancer vaccine.

(Step S10) First, a cancer tissue is excised from a target patient by surgery by a doctor, and a DNA/RNA sample is extracted from the cancer tissue or a tissue specimen.

(Step S20) Next, the DNA/RNA sample is transported and stored, for example, at a vendor. Here, the vendor has a next generation sequencer.

(Step S30) Next, the genome of the DNA/RNA sample is read by the next generation sequencer, and synthesis DNA/RNA data used to synthesize a mutant peptide (i.e. neoantigen) derived from a genetic mutation of cancer is output.

(Step S40) Next, a recipe of a personalized cancer vaccine is created from the synthesis DNA/RNA data.

(Step S50) Next, the personalized cancer vaccine is manufactured from the created recipe.

(Step S60) Next, the personalized cancer vaccine thus manufactured is transported and stored in a hospital.

(Step S70) A target patient is injected with the personalized cancer vaccine.

Here, if the quality of the synthesis DNA/RNA data deteriorates, it is necessary to restart from the transportation of the sample in Step S20 again, and therefore there is a problem that manufacturing of the personalized cancer vaccine is delayed. In view of this problem, this embodiment provides an information processing system and an information processing method for suppressing deterioration in quality of synthesis DNA/RNA data.

### <First Embodiment: Output Switching>

In this embodiment, sequencers are parallelized, and output data is integrated based on a diagnosis function for diagnosing operation data including quality information for each pre-analysis process and/or output quality data. Here, the quality data includes, for example, at least one of a depth of coverage (target region) (read depth), uniformity of coverage (target region), a GC bias, a transition/transversion (Ti/Tv) ratio, base call quality scores, mapping quality, duplicate read success rate and removal of duplicate reads, first base read success, decline in signal intensity, and strand bias.

In specimen collection, it is preferable to use an FFPE specimen, and warm ischemic time (from stop of blood flow to extraction) and cold ischemic time (from extraction to fixation) are important.

For the quality criteria of the extracted nucleic acid, a ΔCt value (ΔΔCq), a DIN value, a Q-value, DV 200, and the like may be used as the quality of nucleic acid (mainly, the degree of fragmentation is evaluated).

With respect to the quality criteria of a library, an index may be set in advance for performing appropriate quality check after the library is prepared and determining whether to proceed to sequence analysis. As the index, the amount of DNA and the fragment size may be used.

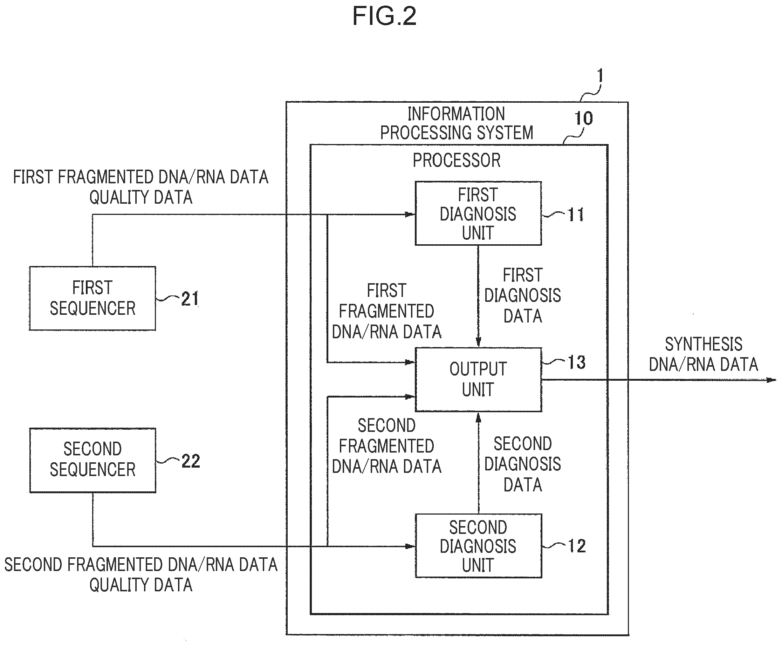

Fig. 2 is a schematic block diagram of the information processing system according to the first embodiment. As illustrated in Fig. 2, an information processing system 1 outputs synthesis DNA/RNA data used for synthesizing a mutated peptide derived from a genetic mutation of cancer, and includes a processor 10. The processor 10 functions as a first diagnosis unit 11, a second diagnosis unit 12, and an output unit 13 by loading a program into a memory (not illustrated) and executing a series of commands included in the program.

A first sequencer 21 is a next generation sequencer, for example, and subjects a sample extracted from a cancer tissue of a target patient to genome sequencing to create first fragmented DNA/RNA data and quality data indicating the quality of the genome sequencing. The first sequencer 21 may have an HLA typing function.

Likewise, a second sequencer 22 is a next generation sequencer, for example, and subjects a sample extracted from a cancer tissue of the target patient to genome sequencing to create second fragmented DNA/RNA data and quality data indicating the quality of the genome sequencing.

The first diagnosis unit 11 acquires the first fragmented DNA/RNA data and the quality data from the first sequencer 21. The first diagnosis unit 11 diagnoses the quality of the sequence of the first fragmented DNA/RNA data, which the first sequencer has output for the sample extracted from the tissue of the target patient, using the quality data output from the first sequencer, and determines at least one first diagnosis data indicating the diagnosis result.

The second diagnosis unit 12 acquires the second fragmented DNA/RNA data and the quality data from the second sequencer 22. The second diagnosis unit 12 diagnoses the quality of the sequence of the second fragmented DNA/RNA data, which the second sequencer has output for the sample extracted from the tissue of the target patient, using the quality data output from the second sequencer, and determines at least one second diagnosis data indicating the diagnosis result.

The output unit 13 acquires the first fragmented DNA/RNA data from the first sequencer 21, the second fragmented DNA/RNA data from the second sequencer 22, the first diagnosis data from the first diagnosis unit 11, and the second diagnosis data from the second diagnosis unit 12. The output unit 13 outputs the synthesis DNA/RNA data from the first fragmented DNA/RNA data and the second fragmented DNA/RNA data based on the first diagnosis data and the second diagnosis data.

This configuration increases the possibility of outputting high-quality synthesis DNA/RNA data for synthesizing a mutated peptide, and suppresses repetition of the process due to deterioration in quality of synthesis DNA/RNA data, so that delay in manufacturing of a personalized cancer vaccine can be suppressed.

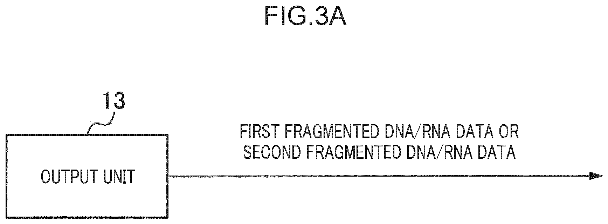

Fig. 3A is a schematic diagram illustrating processing of the output unit according to the first embodiment. In the first embodiment, as illustrated in Fig. 3A, the output unit 13 switches an output between the first fragmented DNA/RNA data and the second fragmented DNA/RNA data based on the first diagnosis data and the second diagnosis data, and outputs the selected output as the synthesis DNA/RNA data. More specifically, the output unit 13 determines which of the first fragmented DNA/RNA data and the second fragmented DNA/RNA data is appropriate based on the first diagnosis data and the second diagnosis data, and switches the output between them.

Specifically, for example, the output unit 13 may output only fragmented DNA/RNA data in which the number of excellent indexes is larger than a set threshold among the indexes set as the multiple indexes of the diagnosis data. According to this configuration, fragmented DNA/RNA data having a large number of excellent indexes is output for synthesizing a mutated peptide, so that repetition of the process due to deterioration in quality of synthesis DNA/RNA data is suppressed, and thus delay in manufacturing of a personalized cancer vaccine can be suppressed.

### <First Modification of First Embodiment: Output Mixing>

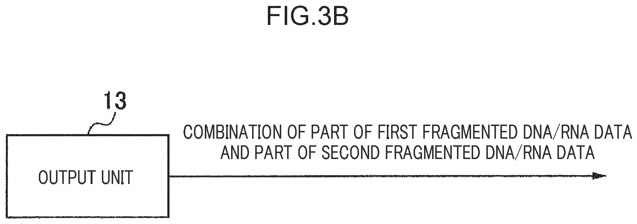

Fig. 3B is a schematic diagram illustrating processing of the output unit according to the first modification of the first embodiment. As illustrated in Fig. 3B, the output unit 13 may combine a part of the first fragmented DNA/RNA data and a part of the second fragmented DNA/RNA data based on the first diagnosis data and the second diagnosis data, and output the combined data as the synthesis DNA/RNA data.

Specifically, for example, when each fragmented DNA/RNA data has an index, the output unit 13c may output a predetermined number of fragmented DNA/RNA data in descending order of excellence of the indexes.

Alternatively, for example, the output unit 13c may analyze the frequency of fragmented data included in the output data (i.e., the first fragmented DNA/RNA data and the second fragmented DNA/RNA data) of both the first sequencer 21 and the second sequencer 22, and output the fragmented data in descending order of frequency.

### <Second Modification of First Embodiment>

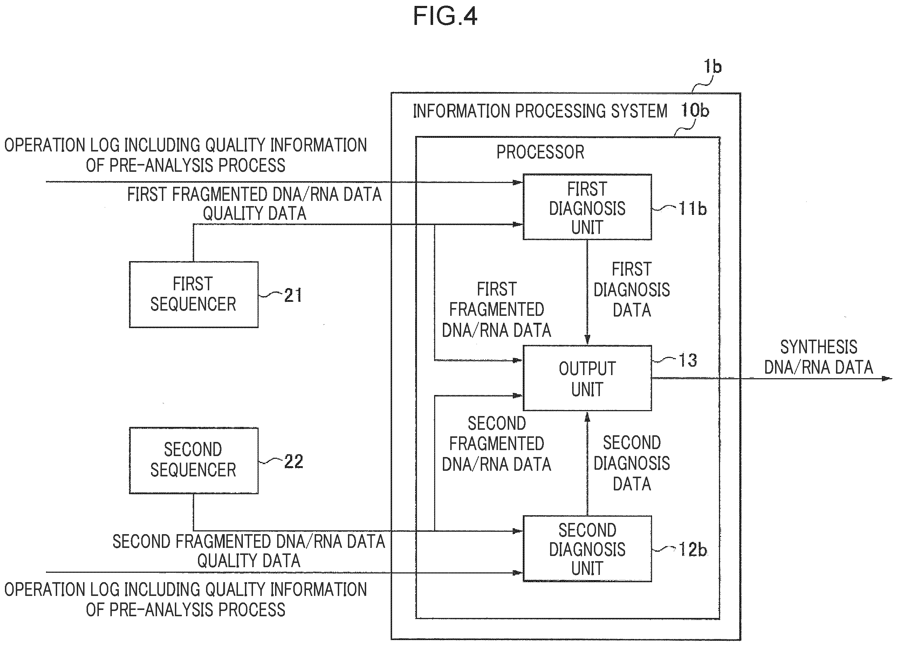

Fig. 4 is a schematic block diagram of the information processing system according to a second modification of the first embodiment. An information processing system 1b of Fig. 4 is different from the information processing system 1 of Fig. 2 in the processing of a processor 10b, and the first diagnosis unit 11 is changed to a first diagnosis unit 11b and the second diagnosis unit 12 is changed to a second diagnosis unit 12b.

The first diagnosis unit 11b acquires an operation log including quality information of a pre-analysis process. The first diagnosis unit diagnoses the quality of the sequence of the first fragmented DNA/RNA data using the quality information of the pre-analysis process, performed before the sequence of the first sequencer 21, in addition to the quality data output from the first sequencer 21, and determines first diagnosis data. Specifically, for example, the first diagnosis unit 11b may diagnose that the quality is good in a case where the quality data satisfies first criteria and the quality information satisfies second criteria, and may diagnose that the quality is poor in other cases and determine the first diagnosis data indicating the diagnosis result.

Likewise, the second diagnosis unit 12b acquires an operation log including quality information of a pre-analysis process. The second diagnosis unit 12b diagnoses the quality of the sequence of the second fragmented DNA/RNA data using the quality information of the pre-analysis process, performed before the sequence of the second sequencer 22, in addition to the quality data output from the second sequencer 22, and determines the second diagnosis data. Specifically, for example, the second diagnosis unit 12b may diagnose that the quality is good in a case where the quality data satisfies the first criteria and the quality information satisfies the second criteria, and may diagnose that the quality is poor in other cases and determine the first diagnosis data indicating the diagnosis result.

### <Third Modification of First Embodiment>

Note that, the output unit 13 may limit the amount of synthesis DNA/RNA data to be output by filtering double output data, made by the combination of the first fragmented DNA/RNA data and the second fragmented DNA/RNA data, based on the first diagnosis data and the second diagnosis data. This filter may use at least one or more of statistical values such as a limitation in number, a size of a diagnosis data value for each read, and an overlap of reads.

This configuration increases the possibility of outputting high-quality synthesis DNA/RNA data for synthesizing a mutated peptide, and suppresses repetition of the process due to deterioration in quality of synthesis DNA/RNA data, so that delay in manufacturing of a personalized cancer vaccine can be suppressed.

### <Second Embodiment: Output Coupling>

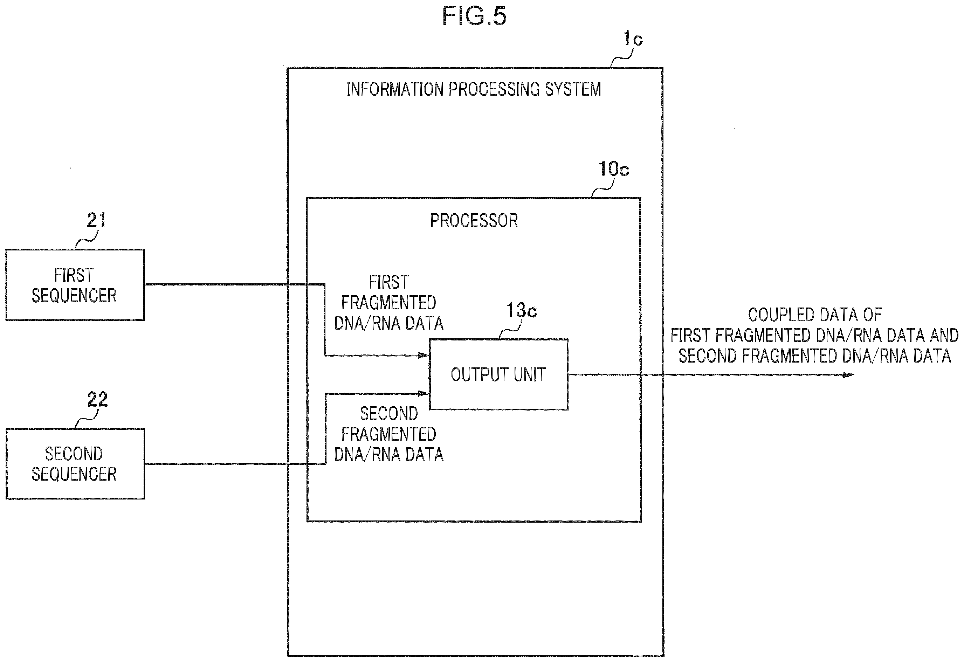

Fig. 5 is a schematic block diagram of an information processing system according to the second embodiment. As illustrated in Fig. 5, an information processing system 1c includes a processor 10c. The processor 10c functions as an output unit 13c by loading a program into a memory (not illustrated) and executing a series of commands included in the program. The output unit 13c couples the first fragmented DNA/RNA data, which the first sequencer has output for the sample extracted from the tissue of the target patient, to the second fragmented DNA/RNA data, which the second sequencer has output for the sample extracted from the tissue of the target patient, and outputs the coupled data as synthesis DNA/RNA data.

Specifically, for example, in a case where the multiple indexes of the diagnosis data exceed the threshold, the output unit 13c may couple both data and output the coupled data as double data.

Alternatively, for example, the output unit 13c may compare both output data, and in a case where their difference is small, may couple both data and output the coupled data as double data.

In addition, in a case where outputting the synthesis DNA/RNA data, the output unit 13c may output diagnosis data including the order of priority of the mutated peptide (neoantigen) together with the synthesis DNA/RNA data. The order of priority may be determined based on the quality information and/or the quality data, and may be set in such a way that the higher the quality, the higher the order of priority. This configuration prioritizes high-quality synthesis DNA/RNA data for synthesizing a mutated peptide, and thus suppresses repetition of the process due to deterioration in quality of synthesis DNA/RNA data, so that delay in manufacturing of a personalized cancer vaccine can be suppressed.

Note that, although the description has been given in the first and second embodiments of the case where the number of parallels is two, the number of parallels may be three or more, and the number of parallels may be determined by the amount of samples collected in surgery.

### <Third Embodiment: Majority Decision>

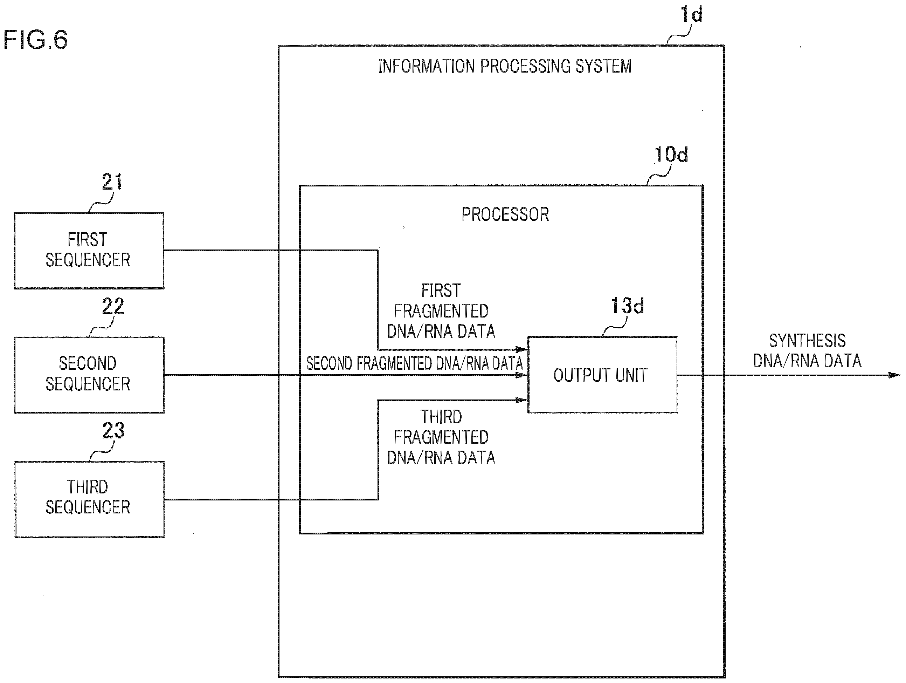

Fig. 6 is a schematic block diagram of an information processing system according to the third embodiment. As illustrated in Fig. 6, an information processing system 1d includes a processor 10d. The processor 10d functions as an output unit 13d by loading a program into a memory (not illustrated) and executing a series of commands included in the program.

The output unit 13d acquires the first fragmented DNA/RNA data from the first sequencer 21. In addition, the output unit 13d acquires the second fragmented DNA/RNA data from the second sequencer 22. Further, the output unit 13d acquires third fragmented DNA/RNA data from a third sequencer 23.

The output unit 13d determines the synthesis DNA/RNA data by carrying out a majority decision between bases of the corresponding order of the first fragmented DNA/RNA data which the first sequencer has output for the sample extracted from the tissue of the target patient, the second fragmented DNA/RNA data which the second sequencer has output for the sample extracted from the tissue of the target patient, and the third fragmented DNA/RNA data which the third sequencer has output for the sample extracted from the tissue of the target patient, and determining the bases of the respective orders.

This configuration increases the accuracy of synthesis DNA/RNA data used for synthesizing a mutated peptide, so that delay in manufacturing of a personalized cancer vaccine can be suppressed.

Note that, not only cancer vaccines but also autoimmune diseases may be treated with similar neoantigens, and therefore a similar embodiment can be employed for personalized medical care of autoimmune diseases.

Note that, at least a part of the information processing system 1 described in the above embodiment may be configured by hardware or software. In a case where the information processing system 1 is configured by software, a program for implementing at least a part of functions of the information processing system 1 may be stored in a computer-readable recording medium, and read and executed by a computer. The recording medium is not limited to a removable recording medium such as a magnetic disk or an optical disk, and may be a fixed recording medium such as a hard disk device or a memory.

Alternatively, a program for implementing at least a part of functions of the information processing system 1 may be distributed via a communication line (including wireless communication) such as the Internet. Further, the program may be distributed via a wired or wireless line such as the Internet or stored in a recording medium in an encrypted, modulated, or compressed state.

Furthermore, the information processing system 1 may be caused to function by one or multiple information devices. In a case where multiple information devices are used, the function may be implemented as at least one means of the information processing system 1 by setting a computer as one of the information devices and causing the computer to execute a predetermined program.

In the invention of the method, all the processes (steps) may be implemented by automatic control by a computer. In addition, while causing a computer to perform each process, process progress control may be performed manually. Furthermore, at least a part of all the steps may be performed manually.

As described above, the present invention is not limited to the above-described embodiments, and can be embodied by modifying the constituents without departing from the gist of the present invention in the implementation stage. In addition, various inventions can be formed by appropriately combining multiple constituents disclosed in the above embodiments. For example, some of the constituents may be deleted from all the constituents illustrated in the embodiments. Furthermore, the constituents in the different embodiments may be appropriately combined.

### Reference Signs List

1, 1b, 1c, 1d INFORMATION PROCESSING SYSTEM
10, 10b, 10c, 10d PROCESSOR
11, 11b FIRST DIAGNOSIS UNIT
12, 12b SECOND DIAGNOSIS UNIT
13, 13c, 13d OUTPUT UNIT
21 FIRST SEQUENCER
22 SECOND SEQUENCER
23 THIRD SEQUENCER

## Claims

1. An information processing system that outputs synthesis DNA/RNA data used for synthesizing a mutated peptide derived from a genetic mutation of cancer, the information processing system comprising:
a first diagnosis unit that diagnoses quality of a sequence of first fragmented DNA/RNA data, which a first sequencer has output for a sample extracted from a tissue of a target patient, using quality data output from the first sequencer, and determines at least one first diagnosis data indicating a diagnosis result;
a second diagnosis unit that diagnoses quality of a sequence of second fragmented DNA/RNA data, which a second sequencer has output for the sample extracted from the tissue of the target patient, using quality data output from the second sequencer, and determines at least one second diagnosis data indicating a diagnosis result; and
an output unit that outputs the synthesis DNA/RNA data from the first fragmented DNA/RNA data and the second fragmented DNA/RNA data based on the first diagnosis data and the second diagnosis data.

2. The information processing system according to claim 1, wherein
the first diagnosis unit diagnoses the quality of the sequence of the first fragmented DNA/RNA data using quality information obtained by a pre-analysis process, performed before the sequence of the first sequencer, in addition to the quality data output from the first sequencer, and determines the first diagnosis data, and
the second diagnosis unit diagnoses the quality of the sequence of the second fragmented DNA/RNA data using quality information obtained by a pre-analysis process, performed before the sequence of the second sequencer, in addition to the quality data output from the second sequencer, and determines the second diagnosis data.

3. The information processing system according to claim 1 or 2, wherein
the output unit switches an output between the first fragmented DNA/RNA data and the second fragmented DNA/RNA data based on the first diagnosis data and the second diagnosis data, and outputs selected output as the synthesis DNA/RNA data.

4. The information processing system according to claim 1 or 2, wherein
the output unit combines a part of the first fragmented DNA/RNA data and a part of the second fragmented DNA/RNA data based on the first diagnosis data and the second diagnosis data, and outputs combined data as the synthesis DNA/RNA data.

5. An information processing system that outputs synthesis DNA/RNA data used for synthesizing a mutated peptide derived from a genetic mutation of cancer, the information processing system comprising
an output unit that couples first fragmented DNA/RNA data, which a first sequencer has output for a sample extracted from a tissue of a target patient, to second fragmented DNA/RNA data, which a second sequencer has output for the sample extracted from the tissue of the target patient, and outputs coupled data as synthesis DNA/RNA data.

6. The information processing system according to claim 5, wherein,
in a case where outputting the synthesis DNA/RNA data, the output unit outputs diagnosis data including an order of priority of the mutated peptide together with the synthesis DNA/RNA data.

7. An information processing system that outputs synthesis DNA/RNA data for synthesizing a mutated peptide derived from a genetic mutation of cancer, the information processing system comprising:
an output unit that determines the synthesis DNA/RNA data by determining bases of respective orders by carrying out a majority decision between bases of a corresponding order of first fragmented DNA/RNA data which a first sequencer has output for a sample extracted from a tissue of a target patient, second fragmented DNA/RNA data which a second sequencer has output for the sample extracted from the tissue of the target patient, and third fragmented DNA/RNA data which a third sequencer has output for the sample extracted from the tissue of the target patient.

8. An information processing method of outputting synthesis DNA/RNA data for synthesizing a mutated peptide derived from a genetic mutation of cancer, the information processing method comprising:
diagnosing quality of a sequence of first fragmented DNA/RNA data, which a first sequencer has output for a sample extracted from a tissue of a target patient, using quality data output from the first sequencer;
determining at least one first diagnosis data indicating a diagnosis result;
diagnosing quality of a sequence of second fragmented DNA/RNA data, which a second sequencer has output for the sample extracted from the tissue of the target patient, using quality data output from the second sequencer;
determining at least one second diagnosis data indicating a diagnosis result; and
outputting the synthesis DNA/RNA data from the first fragmented DNA/RNA data and the second fragmented DNA/RNA data based on the first diagnosis data and the second diagnosis data.

9. An information processing method of outputting synthesis DNA/RNA data for synthesizing a mutated peptide derived from a genetic mutation of cancer, the information processing method comprising:
coupling first fragmented DNA/RNA data, which a first sequencer has output for a sample extracted from a tissue of a target patient, to second fragmented DNA/RNA data, which a second sequencer has output for the sample extracted from the tissue of the target patient; and
outputting coupled data as synthesis DNA/RNA data.

10. An information processing method of outputting synthesis DNA/RNA data used for synthesizing a mutated peptide derived from a genetic mutation of cancer, the information processing method comprising:
determining the synthesis DNA/RNA data by determining bases of respective orders by carrying out a majority decision between bases of a corresponding order of first fragmented DNA/RNA data which a first sequencer has output for a sample extracted from a tissue of a target patient, second fragmented DNA/RNA data which a second sequencer has output for the sample extracted from the tissue of the target patient, and third fragmented DNA/RNA data which a third sequencer has output for the sample extracted from the tissue of the target patient.
